# EUROPEAN PATENT SPECIFICATION

(11) **EP 2 247 269 B1**
(45) Date of publication and mention of the grant of the patent: **24.08.2011**
(21) Application number: 09703737.8
(22) Date of filing: 14.01.2009
(51) Int. Cl.: A61F 2/90, B05C 13/02

(54) **STENT FOR DELIVERING A THERAPEUTIC AGENT FROM A SIDE SURFACE OF A STENT STRUT**
STENT ZUM AUSBRINGEN EINES THERAPEUTISCHEN MITTELS VON EINER SEITENFLÄCHE EINER STENTSTREBE
STENT APTE À DÉLIVRER UN AGENT THÉRAPEUTIQUE À PARTIR D'UNE SURFACE LATÉRALE D'UN SUPPORT DE STENT

(30) Priority: 24.01.2008 US 23142 P
(43) Date of publication of application: 10.11.2010
(73) Proprietor: Boston Scientific Scimed, Inc., Maple Grove, MN 55311-1566 (US)
(72) Inventor: MEYER, Michael P., Richfield, Minnesota 55423 (US); MCGOVERN, James E., Maple Grove, MN 55369 (US); GREGORICH, Daniel, Plymouth, MN 55442 (US); SORENSON, Shawn, Maple Grove, Minnesota 55311 (US); FOSS, Aaron, Plymouth, Minnesota 55447 (US)
(74) Representative: Hauck Patent- und Rechtsanwälte
(86) International application number: PCT/US2009/030936
(87) International publication number: WO 2009/094270

(56) References cited:
- WO-A-02/47581
- WO-A-90/04982
- WO-A-2008/034066
- US-A- 6 071 305
- US-A1- 2007 112 421
- US-B1- 7 135 038

## Description

### CROSS-REFERENCE TO RELATED APPLICATIONS

This application claims priority from U.S. Provisional Application No. 61/023,142, filed January 24, 2008,

### FIELD OF INVENTION

Described herein are implantable medical devices, such as implantable or intravascular stents, for delivering a therapeutic agent, and methods for making such medical devices. In one embodiment, the medical device comprises a stent having a plurality of struts, at least one of which has a cavity disposed therein. A therapeutic agent is delivered from the cavity through an opening in a strut surface. In another embodiment, the medical device is a stent having a coating disposed on the side surface(s) of at least one strut for deliver of a therapeutic agent from the coating.

### BACKGROUND

Medical devices have been used to deliver therapeutic agents locally to the body tissue of a patient. For example, stents having a coating containing a therapeutic agent, such as an anti-restenosis agent, have been used in treating or preventing restenosis. Currently, such medical device coatings include a therapeutic agent alone of a combination of a therapeutic agent and a polymer. Some polymer coating compositions, however, do not actually adhere to the surface of the medical device. In order to ensure that the coating compositions remain on the surface, the area of the medical device that is coated, such as a stent strut, is encapsulated with the coating composition. However, since the polymer does not adhere to the medical device, the coating composition is susceptible to deformation and damage during loading, deployment and implantation of the medical device. Any damage to the polymer coating may alter the therapeutic agent release profile and can lead to an undesirable increase or decrease in the therapeutic agent release rate.

Furthermore, by encapsulating a stent strut with a coating comprising a therapeutic agent, an amount of the therapeutic agent greater than the desired amount may be delivered. For instance, if the therapeutic agent is an anti-restenosis agent, by applying a coating containing such an agent to all surfaces of the strut, including the luminal surface, may result in the unnecessary delivery of the anti-restenosis agent to the bloodstream.

US 7135038 refers to an implantable stent having struts with narrowed portions. Those narrowed portions having a coating disposed thereon for the local delivery of a drug.

Accordingly, there is a need for medical devices that can release an effective amount of a therapeutic agent in a desired manner while avoiding the disadvantages of current medical devices for delivering a therapeutic agent. Additionally, there is a need for methods of making such medical devices.

### SUMMARY

These and other objectives are addressed by the medical devices described herein. In one embodiment, the medical device comprises an implantable stent, which comprises a stent sidewall structure comprising a plurality of struts and gaps in the sidewall structure. At least one strut comprises an abluminal surface, a luminal surface, and a first side surface. The strut comprises a first material. Also, the at least one strut comprises at least one cavity disposed therein. The cavity has a first opening that is in fluid communication with the abluminal surface and a second opening that is in fluid communication with the first side surface. A therapeutic agent can be disposed in the cavity. Furthermore, a coating is disposed over at least a portion of the first opening of the cavity that is in fluid communication with the abluminal surface. The second opening of the cavity, which is in fluid communication with the side surface, is at least partially exposed.

In another embodiment, the medical device comprises an implantable stent that comprises a stent sidewall structure comprising a plurality of struts and gaps in the sidewall structure. At least one strut comprises an abluminal surface, a luminal surface, and a first side surface. Also, the at least one strut comprises at least one cavity disposed therein, in which the cavity has a first opening that is in fluid communication with the first side surface and the first opening of the cavity is at least partially exposed. The cavity does not have any opening that is in fluid communication with the abluminal surface or the luminal surface. Also a therapeutic agent is disposed in the cavity.

In addition, in one embodiment, the medical device comprises an implantable stent that comprises a stent sidewall structure comprising a plurality of struts and gaps in the sidewall structure. At least one of the struts comprises an abluminal surface, a luminal surface, and a first side surface. A first coating is disposed on at least a portion of the first side surface and the abluminal and luminal surfaces are substantially free of any coating.

Also described herein are methods for making medical devices. In one embodiment, the method is one for making an implantable stent. The method comprises the step of providing a tube having a tubular wall having a longitudinal axis, in which the tubular wall comprises an abluminal surface and luminal surface. There is at least one groove disposed in the abluminal surface of the tubular wall and the groove does not extend through the tubular wall to the luminal surface. A therapeutic agent is disposed in at least a portion of the groove. A coating is disposed over at least a portion of the abluminal surface such that at least a portion of the therapeutic agent disposed in the groove is covered by the coating. A stent is formed from the tube that has the coating disposed on the abluminal surface. The stent has a stent sidewall structure comprising a plurality of struts and gaps, wherein at least one of the struts comprises an abluminal surface, a luminal surface, and a first side surface. Also, the strut has a cavity that comprises a portion of the groove containing the therapeutic agent, and the cavity that comprises a portion of the groove containing the therapeutic agent, and the cavity has an opening, which is at least partially exposed, that is in fluid communication with the first side surface. Alternatively, instead of disposing a therapeutic agent in the groove, a filler material is disposed in the groove. After the stent is formed, the filler material is removed from the cavity and a therapeutic agent is disposed in the cavity.

In another embodiment, the method for making an implantable stent comprises providing a flat sheet of a material having a sheet wall. The sheet wall comprises a first surface and second surface. The method comprises disposing at least one groove in the first surface of the sheet wall, wherein the groove does not extend through the sheet wall to the second surface. A therapeutic agent is then disposed in at least a portion of the groove. A coating is disposed over at least a portion of the first surface such that at least a portion of the therapeutic agent disposed in the groove is covered by the coating. A sidewall structure is formed from the sheet, having the coating disposed on the first surface, by removing portions of the coated sheet. The a sidewall structure comprising a plurality of struts and gaps, wherein at least one of the struts comprises a top surface, a bottom surface, a first side surface and a cavity. The cavity comprises a portion of the groove containing the therapeutic agent, and an opening that is in fluid communication with the first side surface. The opening is at least partially exposed. Instead of disposing a therapeutic agent in the groove, a filler material can be disposed in the groove. After the sidewall structure is formed, the filler material is removed from the cavity and a therapeutic agent is disposed in the cavity.

In another method for making an implantable stent, the method comprises providing a stent having a stent sidewall structure comprising a plurality of struts and gaps in the sidewall structure. There is at least one strut comprises an abluminal surface, a luminal surface, and a first side surface. Also, at least one vacity is disposed in the strut and the cavity has a first opening that is in fluid communication with the first side surface and a second opening that is in fluid communication with the abluminal surface or the luminal surface. The method further comprises disposing a therapeutic agent in the cavity and applying a material over the second opening and any other opening so that the cavity is not in fluid communication with the abluminal surface or luminal surface. However, the first opening is at least partially exposed.

In a further embodiment, the method for making an implantable stent comprises providing a stent having a stent sidewall structure comprising a plurality of struts and gaps in the sidewall structure. At least one strut comprises an abluminal surface, a luminal surface, and a first side surface. There is at least one cavity disposed in the strut that has a first opening that is in fluid communication with the first side surface. The cavity is not in fluid communication with the abluminal surface or the luminal surface. The method further comprises disposing a therapeutic agent in the cavity and allowing the first opening to be at least partially exposed.

Moreover, in another embodiment, the method for making an implantable stent comprises providing a stent having a stent sidewall structure comprising a plurality of struts and gaps in the sidewall structure, wherein at least one strut comprises an abluminal surface, a luminal surface, and a first side surface. The stent is disposed on a mandrel. A coating composition comprising a therapeutic agent is applied onto a flat surface. The stent disposed on the mandrel is rolled in the coating composition applied to the flat surface to apply the coating composition to the first side surface of the strut. Steps are taken to ensure that the abluminal surface and the luminal surface are substantially free of the coating composition.

### BRIEF DESCRIPTION OF THE DRAWINGS

The embodiments described herein will be explained with reference to the following drawings.
**Figure 1** shows a perspective view of an example of a medical device having a sidewall structure comprising a plurality of struts and gaps or opening and at least one cavity disposed in at least one strut.
**Figure 2A** shows a perspective view of a strut of one embodiment of a medical device, such as a stent.
**Figure 2B** shows a portion of the strut of **Figure 2A** located between lines **A-A** and **B-B** when the portion is placed in a vessel.
**Figure 2C** shoes a cross-sectional view of the strut portion of **Figure 2B** at line **D-D.**
**Figure 3A-3G** shows perspective view of struts from certain embodiments, in which the struts comprise a cavity.
**Figure 4A-4M** show perspective views of struts from additional embodiments, in which the struts comprise a cavity.
**Figure 5A-5G** show cross-sectional views of struts from certain embodiments, in which the struts comprise a cavity.
**Figure 6** shows a perspective view of a strut of another embodiment.

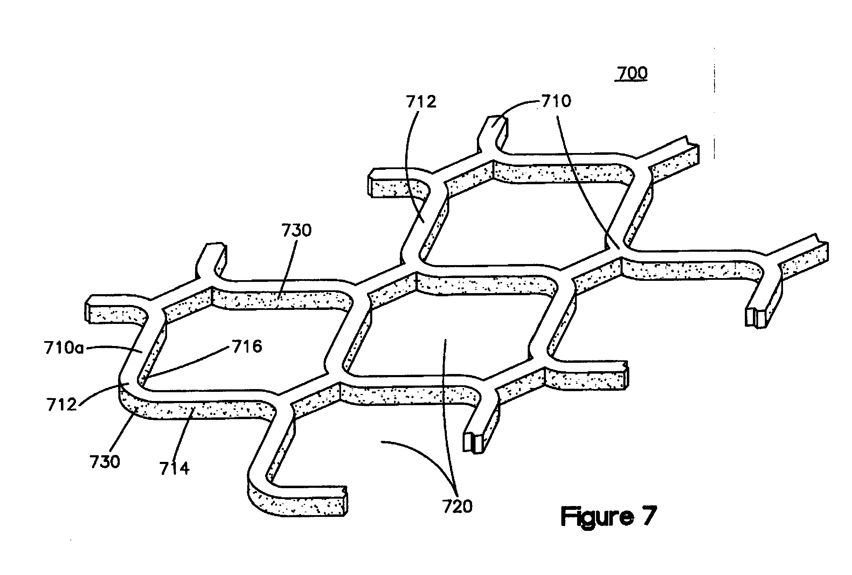

**Figure 7** shows a perspective view of an embodiment of a stent in which a coating composition is disposed on a side surface of a strut of the stent.
**Figure 8A-8F** show one embodiment of a method of making a stent.
**Figure 9** shows a step in an embodiment of a method of making a stent.
**Figure 10A-10F** show another embodiment of a method of making a stent.
**Figure 11** shows a step in an embodiment of a method of making a stent.
**Figure 12A-12C** show another embodiment of a method of making a stent.
**Figure 13A-13B** show yet another embodiment of a method of making a stent.

### DETAILED DESCRIPTION THE MEDICAL DEVICES

In one embodiment, the medical device is a stent comprising a stent sidewall structure, which comprises a plurality of struts and gaps in the sidewall structure. At least one strut comprises a first material. Also, the at least one strut comprises an abluminal surface, a luminal surface, and a first side surface. The abluminal surface of the strut is the surface that faces away from the lumen or towards the lumen wall, *e.g*., a wessel wall, when the stent is implanted in a lumen. The luminal surface of the strut is the surface that faces toward the lumen or away from the lumen wall when the stent is implanted in a lumen. The side surface of the strut is a surface that is disposed between the abluminal and luminal surfaces of the strut. In some instances, where the strut is formed by being cut from a material, such as a metal tube, the side surface can be cut-surface, *i.e.* the surface that is formed when the strut is cut from the material. Furthermore, there is at least one cavity disposed in the at least one strut, wherein the cavity comprises an opening that is in fluid communication with a side surface of the strut. A therapeutic agent can be disposed in the cavity. This agent can be released from the cavity through the opening.

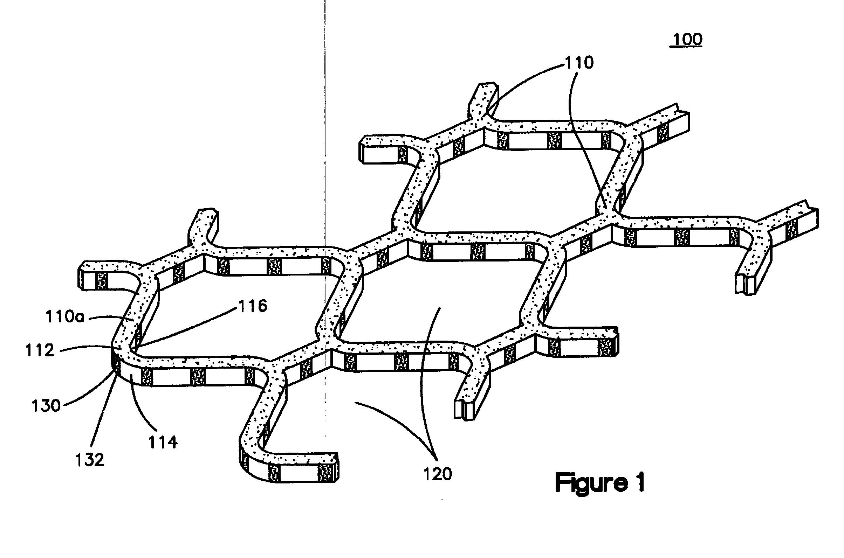

**Figure 1** shows an embodiment of a portion of a sidewall structure **100** of a stent. The sidewall structure **100** comprises struts **110** and gaps **120.** At least one of the struts **110a** has an abluminal surface **112** and a first side surface **114** and a second side surface **116.** Also, the at least one strut **110a** comprises a cavity **130** disposed therein. A therapeutic agent can be disposed in the cavity. The cavity **130** has an opening **132** that is in fluid communication with the first side surface **114.** The cavity **130** can also have another opening (not shown) that is in fluid commmuication with the second side surface **116,** the abluminal surface **112** or the luminal surface (not shown) of the strut **110a.** In this embodiment, the opening **132** is at least partially exposed, *i.e*., not covered by a material. In some embodiments, the entire opening is exposed or a part of the opening is exposed.

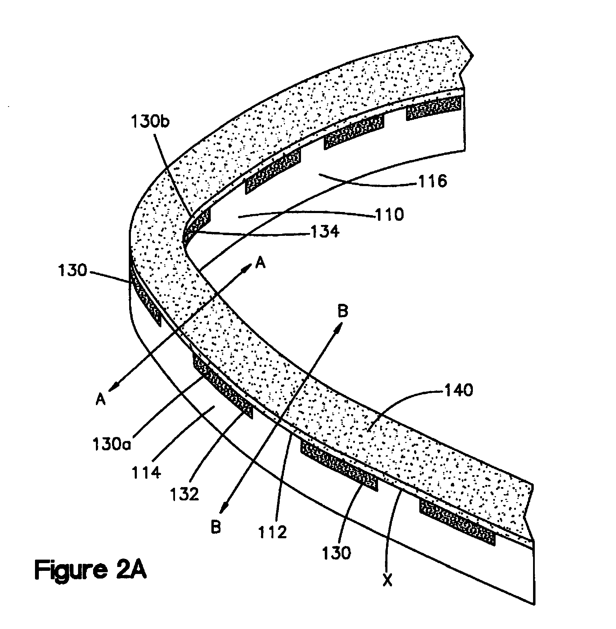

Shown is **Figure 2A** is one embodiment of a strut. In this embodiment, a coating is disposed over an opening of a cavity that is disposed in the strut. More specifically, in this embodiment, the strut **110** has a number of cavities **130** disposed therein. At least one of the cavities **130a** has a first opening **132** that is in fluid communication with a side surface **114** of the strut **110.** The cavity **130a** also has a second opening (not shown) that is in fluid communication with the abluminal surface **112** of the strut **110.** Also, the cavity **130a** can have a third opening (not shown) that is in fluid communication with the second side surface **116** of the strut **110,** like opening **134** of cavity **130b.** As described below, a cavity can have various configurations. Also, cavities having different configurations can be disposed in the same or different struts. Furthermore, as described below, the opening can have various shapes or configurations and opening of different shapes or configurations can be disposed in the same or different struts.

In this embodiment, a coating **140** is disposed over at least a portion of the opening of the cavity **130a** that is in fluid communication with the abluminal surface **112.** In some embodiments, the coating is disposed over the entire opening of the cavity that is in fluid communication with the abluminal surface. In others, the coating is disposed over less than the entire opening. In addition, in this embodiment, the coating **140** is disposed over a portion of the abluminal surface **112** of the strut **110,** at for example position **X**.

The coating can comprise the same material as the strut. Alternatively, the coating can comprise a material that is different from the material of the strut. In certain embodiments, the coating comprises a polymer, a metal, an oxide, a ceramic, or different combination or composites of such materials (*e.g*., a composite of a ceramic and a polymer). In certain embodiments, the coating can comprise a radiopaque material.

In some instances, the coating is substantially free of any polymer, *i.e.*, no polymer is intentionally added to the coating material. In other embodiments, the coating can comprise a polymer that modulates release of the therapeutic agent from the cavity through the coating. Also, in some embodiments, the coating comprises a material that prevents the release of the therapeutic agent from the cavity through the coating. In the alternative, the coating comprises a material have a plurality of pores therein that allows for the release of the therapeutic agent from the cavity through the coating. In the alternative, the coating comprises a material have a plurality of pores therein that allows for the release of the therapeutic agent from the cavity through the coating. Moreover, in come embodiments, the coating can comprise a therapeutic agent, which can be the same as or different from the therapeutic agent disposed in the cavity or cavities. For example, the coating can comprise a polymer and a first therapeutic agent. In such an embodiment, the first therapeutic agent is released from the abluminal surface of the strut while a second therapeutic agent in the cavity is released from the cut face.

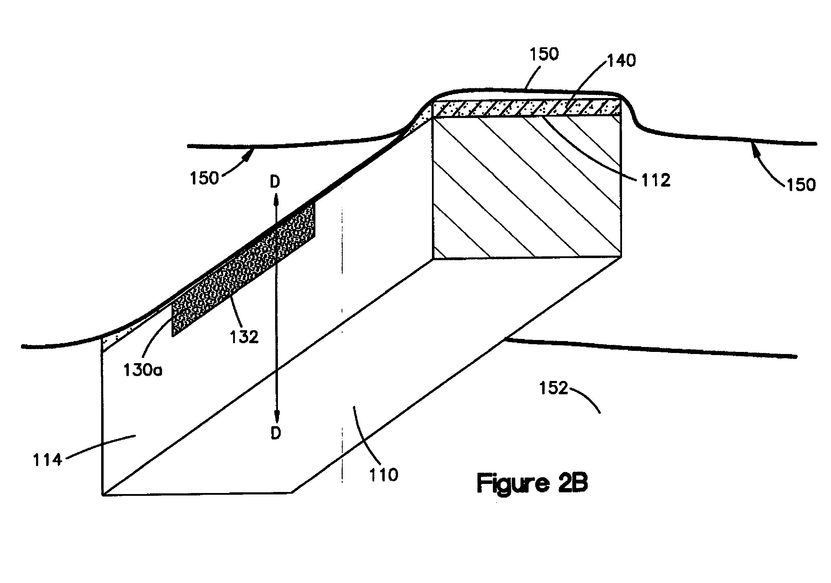

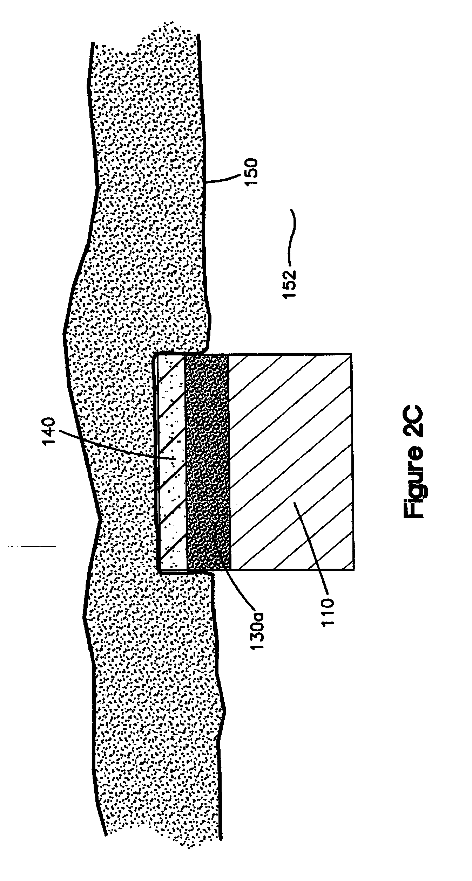

**Figure 2B** shows the portion of the strut **110** situated between lines **A-A** and **B-B** in **Figure 2A****.** The portion of the stent is shown as being inserted or implanted in a body lumen, such as a blood vessel **152.** As shown in **Figure 2B****,** the coating **140** is placed in contact with the vessel wall **150.** Since the opening **132** of the cavity **130a** is at least partially exposed, the therapeutic agent disposed in the cavity **130a** can be released from the cavity **130a** into the vessel **152** or vessel wall **150.** **Figure 2C** shows a cross-sectional view of the strut shown in **Figure 2B** at line **D-D.** As shown in this figure, the coating **140** is disposed over the opening of the cavity **130a** that is in fluid communication with the abluminal surface of the strut **110.** Also, as shown in this figure, the first and third openings that are each in fluid communication with a side surface are in contact with at least a portion of the second opening that is in fluid communication with the abluminial surface *i.e.*, there is no material separating at least a portion of the opening.

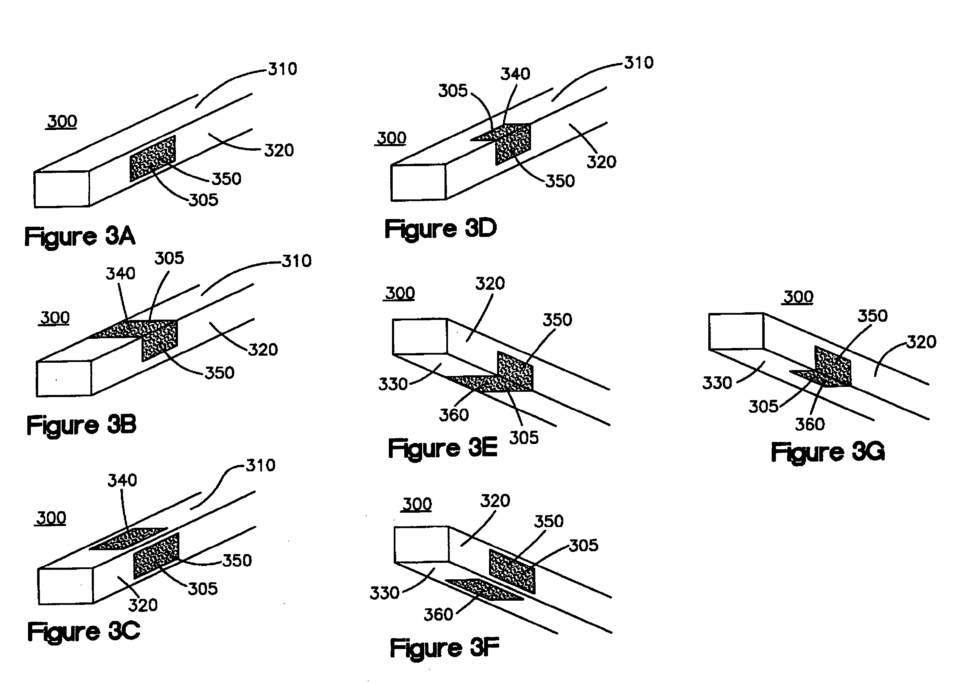

**Figures 3A-3G** show various configurations of openings of cavities **305** that can be disposed in a strut. Although these figures do not show a coating disposed over the opening(s) of the cavities, coatings can be disposed over at least a portion of one or more openings. **Figure 3A** shows an example of a strut **300** having a cavity **305** disposed therein. The cavity **305** has an opening **350** at a single side surface **320.** In this embodiment, the cavity **305** has no other opening that extends through a surface of the strut **300.** In other embodiments, the single opening of the cavity can be present in a different surface of the strut, such as the abluminal surface **310.**

**Figures 3B, 3C** and **3D** show embodiments where the cavity **305** has a first opening **340** that is in fluid communication with the abluminal surface **310** of the strut **300** and a second opening **350** that is in fluid communication with a side surface **320** of the strut **300.** In **Figures 3B** and **3D****,** a least a portion of opening **340** is in contact with a portion of opening 350, *i.e.* there is no material separating the portions of the openings. In **Figure** 3 **C,** the openings **340, 350** are not in contact with each other. Furthermore, in **Figure 3B****,** the opening **340** extends across the entire width of the abluminal surface **310.** In **Figure 3D****,** the opening **340** does not extend across the entire width of the abluminal surface **310.** Also, in **Figures 3B, 3C** and **3D****,** the cavities may or may not have additional openings, like for instance in the luminal surface or other side surface (not shown).

A cavity **305** can also have an opening **360** that is in fluid communication with the luminal surface **330** of the strut **300** as shown in **Figures 3E, 3F** and **3G****.** In **Figures 3E** and **3G****,** a least a portion of opening **360** is in contact with a portion of opening **350.** In **Figure 3F****,** the openings **350, 360** are not in contact with each other. Furthermore, in **Figure 3E****,** the opening **360** extends across the entire width of the luminal surface 330. In **Figure 3G****,** the opening **360** does not extend across the entire width of the luminal surface **330.** Also, in **Figures 3E, 3F** and **3G****,** the cavities may or may not have additional openings, like for instance in the abluminal surface or other side surface (not shown).

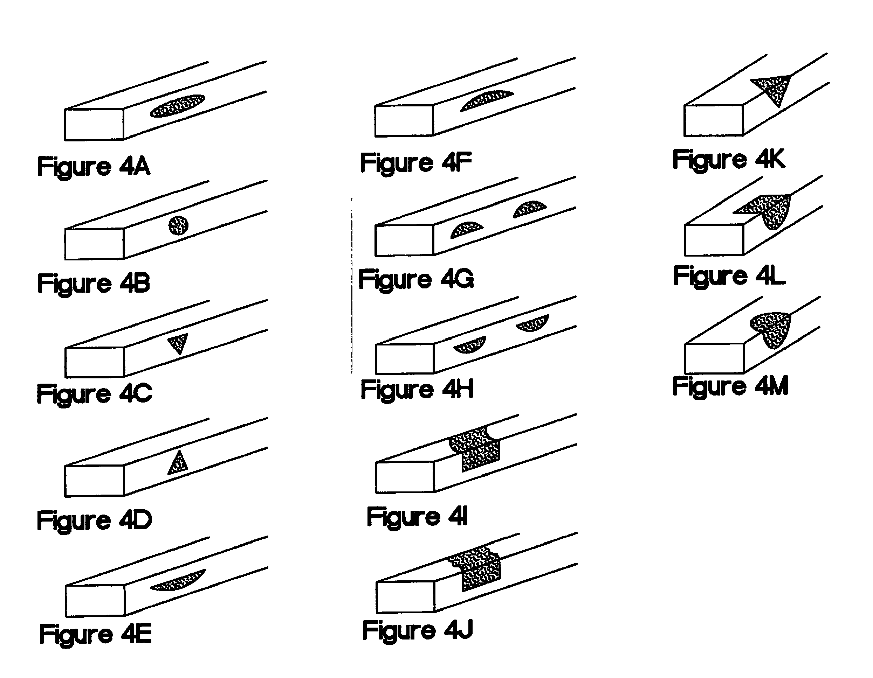

The openings of the cavities may have various shapes and sizes. **Figures 4A-4M** show examples of shapes of openings. **Figure 4A** shows an opening having an oval shape, and **Figure 4B** shows an opening having a circular shape. **Figures 4C** and **4D** show openings having triangular shapes. **Figures 4E** and **4F** show openings having half-oval shapes, and **Figures 4G** and **4H** show openings having semicircular shapes. **Figures 4I-4M** show examples of opening having various shapes in which at least a portion of one opening is in contact with a portion of another opening. Various modifications to the shapes, in addition to those shown and described herein, will become apparent to those skilled in the art.

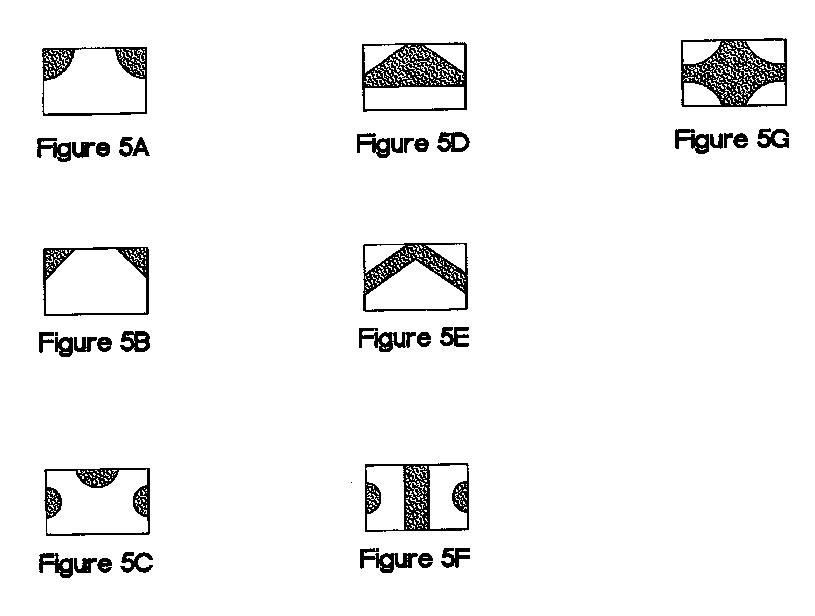

The cavities may have various shapes and sizes. **Figures 5A** to **5G** show cross-sectional views of a number of cavity shapes. Various modifications to the shapes, in addition to those shown and described herein, will become apparent to those skilled in the art.

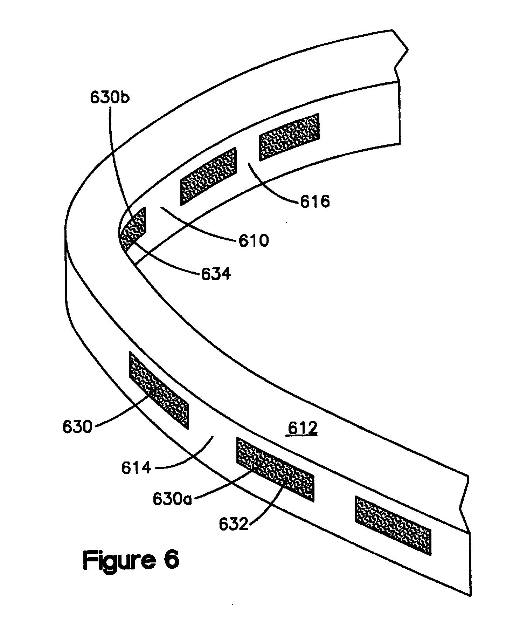

The embodiment shown in **Figures 2A-2C** includes a coating disposed over at least a portion of an opening of a cavity. Other embodiments of struts, such as any of those described above, may not include such a coating. **Figure 6** shows an embodiment that does not include such a coating. In this embodiment, the strut **610** has a number of cavities **630** disposed therein. At least one of the cavities **630a** has an opening **632** that is in fluid communication with a side surface **614** of the strut **610**. The cavity **630a** also has an opening (not shown) that is in fluid communication with the other side surface **616** of the strut **610,** like opening **634** of cavity **630b.** In this embodiment, the openings of the cavities are at least partially exposed. In addition, in this embodiment, the cavity **630a** does not have any openings that are in fluid communication with the abluminal surface **612** or the luminal surface of the strut **610.** Thus, a therapeutic agent that is disposed in the cavity cannot be released through the abluminal or luminal surface of the strut. The therapeutic agent can be released through the openings in the side surfaces of the strut. In some embodiments, a coating can be disposed on one or more surfaces of the strut. The coating can comprise the materials discussed herein.

In yet another embodiment, the medical device comprises a stent having a sidewall structure **700** comprising a plurality of struts **710** and gaps **720** in the sidewall structure **700.** In certain embodiments, the stent is self-expanding stent. At least one of the struts **710,** such as strut **710a,** has an abluminal surface **712,** a luminal surface (not shown) and a first side surface **714** and a second side surface **716.** A coating **730,** which can comprise a polymer and/or a therapeutic agent, is disposed on at least a portion of one or more of the side surfaces **714, 716** of the strut **710.** The abluminal surfaces **712** and luminal surfaces of the struts are substantially free of the coating, *i.e.*, no coating is intentionally disposed on these surfaces. In some embodiments, the abluminal and luminal surfaces are substantially free of any coating.

### Types of Medical Devices

Medical devices suitable for the present embodiments, but are not limited to, those that have a tubular or cylindrical like portion. For example, the tubular portion of the medical device need not be completely cylindrical. The cross-section of the tubular portion can be any shape, such as rectangle, a triangle, etc., not just a circle. Such devices include, but are not limited to, stents, balloon catheters, and grafts. A bifurcated stent is also included among the medical devices which can be fabricated by the methods described herein.

In addition, the tubular portion of the medical device may be a sidewall that may comprise a plurality of struts defining a plurality of openings. The sidewall defines a lumen. The struts may be arranged in any suitable configuration. Also, the struts do not all have to have the same shape or geometric configuration. When the medical device is a stent comprising a plurality of struts, the surface is located on the struts. Each individual strut has an outer surface adapted for exposure to the body tissue of the patient, an inner surface, and at least one side surface between the outer surface and the inner surface.

Medical devices that are particularly suitable for the embodiments described herein include any kind of stent for medical purposes which is known to the skilled artisan. The stents can be intravascular stents that are designed for permanent implantation in a blood vessel of a patient. In certain embodiments, the stent comprises an open lattice sidewall stent structure. In exemplary embodiments, a stent suitable is a coronary stent. Other suitable stents include, for example, vascular stents such as self-expanding stents and balloon expandable stents. Examples of self-expanding stents that can be used are illustrated in United States Patent Nos. 4,655,771 and 4,954,126 issued to Wallsten and 5,061,275 issued to Wallsten et al. Examples of appropriate balloon-expandable stents are shown in United States Patent No. 5,449,373 issued to Pinchaski et al.

In one embodiments, the intravascular stent is generally cylindrical in shape. The stent includes a sidewall structure which comprises a plurality of struts and at least one gap in the sidewall structure. Generally, the gap is disposed between adjacent struts. Also, the sidewall structure may have a first sidewall surface and an opposing second sidewall surface. The first sidewall surface can be an outer or abluminal sidewall surface, which faces a body lumen wall when the stent is implanted, or an inner or luminal sidewall surface, which faces away from the body lumen surface. Likewise, the second sidewall surface can be an abluminal sidewall surface or a luminal sidewall surface. At least one strut comprises an abluminal surface, which forms part of the abluminal surface of the stent, and at least one strut comprises a luminal surface opposite the abluminal surface of the strut, which forms part of the luminal surface of the stent.

In some embodiments, the abluminal surface of the stent sidewall structure comprises at least on cavity and the luminal surface is free of cavities. In other embodiments, the cavity or cavities can be located on a low-stress bearing part of the stent sidewall structure.

When the coatings described herein are applied to a stent having openings in the stent sidewall structure, in certain embodiments, in come embodiments, the coatings conform to the surface of the stent so that the openings in the sidewall stent structure are preserved. *e.g*. the openings are not entirely or partially occluded with coating material.

The framework of suitable stents may be formed through various methods as known in the art. The framework may be welded, molded, laser cut, electroformed, or consist of filaments or fibers which are wound or braided together in order to form a continuous structure.

The medical devices may be fabricated from a metallic material, ceramic material, polymeric or non-polymeric material, or a combination thereof (see Sections 5.1.1.1 to 5.1.1.3 *infra*.). Preferably, the materials are biocompatible. The material may be porous or non-porous, and the porous structural elements can be microporous or nanoporous. Further the coating may be a different material from the substrate or the same material as the substrate.

### Metallic Materials for Medical Devices

In certain embodiments, the medical device comprises a substrate or coating that is metallic. Suitable metallic materials useful for making the substrate or the coating include, but are not limited to, metals and alloys based on titanium (such as nitinol, nickel titanium alloys, thermo memory alloy materials), stainless steel, gold, platinum, iridium, molybdenum, niobium, palladium, chromium, tantalum, nickel, nickel chrome, cobalt, tungsten, or alloys thereof and/or combinations thereof. Examples of alloys include platinum iridium alloys, cobalt-chromium alloys, including cobalt chromium nickel alloys such as Elgiloy^{®} and Phynox^{®}, MP35N alloy, and nickel-titanium alloys, for example, Nitinol. Other metallic materials that can be used to make the medical device include clad composite filaments, such as those disclosed in WO 94/16646.

In some embodiments, the metal is a radiopaque material that makes the medical device visible under X-ray or fluoroscopy. Suitable materials that re radiopaque include, but are not limited to, gold, tantalum, platinum, bismuth, iridium, zirconium, iodine, titanium, barium, silver, tin, alloys of these metals, or a combination thereof.

Furthermore, although a single type of metal can be used to form the substrate, various combinations of metals can also be employed. The appropriate mixture of metals can be coordinated to produce desired effects when incorporated into a substrate.

### Ceramic Materials for Medical Devices

In certain embodiments, the medical device comprises a substrate or a coating which is ceramic. Suitable ceramic materials used for making the substrate or coating include, but are not limited to, oxides, carbides, or nitrides of the transition elements such as those containing titanium, hafnium, iridium, chromium, aluminum, zirconium, transition metals, platinum, tantalum, niobium, tungsten, rhodium, iron, vanadium, nickel, or a combination thereof. Silicon based materials, such as silica, may also be used. Furthermore, although a single type of ceramic can be used to form the substrate, various combinations of ceramics can also be employed. The appropriate mixture of ceramics can be coordinated to produce desired effects when incorporated into a substrate.

### Polymeric Materials for Medical Devices

In certain embodiments, the medical device comprises a substrate or a coating which is polymeric. In other embodiments, the material can be a non-polymeric material. The polymer(s) useful for forming the components of the medical devices should be ones that biocompatible and avoid irritation to body tissue. The polymers can be biostable or bioabsorbable. Suitable polymeric materials useful for making the substrate include, but are not limited to, isobutylene-based polymers, polystyrene-based polymers, polyacrylates, and polyacrylate derivatives, vinyl acetate-based polymers and its copolymers, polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, chitins, or a combination thereof.

Other polymers that are useful as materials for making the substrate include, but are not limited to, Dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates, polyphosphazenes, poly(amino acids), ethylene glycol I dimethacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) co-polymer, polylactic acid, poly(ε-caprolactone), poly(β-hydroxybutyrate), polydioxanone, poly(γ-ethyl glutamate), polyiminocarbonates, poly(ortho ester), polyanhydrides, styrene isobutylene styrene, polyetheroxides, polyvinyl alcohol, polyglycolic acid, polylactic acid, polyamides, poly-2-hydroxy-butyrate, polycaprolactone, poly(lactic-co-clycolic)acid, Teflon, alginate, dextran, chitin, cotton, polyglycolic acid, polyurethane, derivatized versions thereof, (*i.e.*, polymers which have been modified to include, for example, attachment sites or cross-linking groups, *e.g*., arginine-glycine-aspartic acid RGD, in which the polymers retain their structural integrity while allowing for attachment of cells and molecules, such as proteins and/or nucleic acids), or a combination thereof.

The polymers may be dried to increase their mechanical strength. The polymers may then be used as the base material to form a whole or part of the substrate.

Furthermore, although a single type of polymer can be used to form the substrate, various combinations of polymers can also be employed. The appropriate mixture of polymers can be coordinated to produce desired effects when incorporated into a substrate.

### Therapeutic Agents

The term "therapeutic agent" as used herein encompasses drugs, genetic materials, and biological materials and can be used interchangeably with "biologically active material". The term "genetic material" means DNA or RNA, including, without limitation, DNA/RNA encoding a useful protein stated below, intended to be inserted into a human body including viral vectors and non-viral vectors.

The term "biological materials" include cells, yeasts, bacterial, proteins, peptides, cytokines and hormones. Examples for peptides and proteins include vascular endothelial growth factor (VEGF), transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) (*e.g*., BMP-2, BMP-3, BMP-4, BMP-5, BMP-6 (Vgr-1), BMP-7 (PO-1), BMP-8, BMP-9, BMP-10, BMP-11, BMP-12, BMP-14, BMP-15, BMP-16, etc.), matrix metalloproteinase (MMP), tissue inhibitor of matrix metalloproteinase (TIMP), cytokines, interleukin (*e.g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, laminin, glycosaminoglycans, proteoglycans, transferring, cytotactin, cell binding domains (*e.g*., RGD), and tenascin. Examplary BMP's are BMP-2, BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e.g*., endothelial progenitor cells), stem cells (*e.g*., mesenchymal, hematopoietic, neuronal), stromal cells, parenchymal cells, undifferentiated cells, fibroblasts, macrophage, and satellite cells.

Other suitable therapeutic agents include:
- anti-thrombogenic agents such as heparin, heparin derivatives, urokinase, and PPack (dextrophenylalanine praline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, pimecrolimus, sirolimus, zotarolimus, amlodipine and doxazosin;
- anti-inflammatory agents such as glucorticoids, betemethasone, dexamethasone, prednisolone, corticosterone, budesonide, estrogen, sulfasalazine, rosiglitazone, mycophenolic acid and mesalamine;
- anti-neoplastic/anti-proliferative/anti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives, paclitaxel as well as its derivatives, analogs or paclitaxel bound to proteins, *e.g*. Abraxane™;
- anesthetic agents such as lidocaine, bupivacaine, and ropivacaine;
- anit-coagulants such as D-Phe-Pro-Arg chloromethyl ketone, an RGD peptide-containing comound, heparin, antithrombin compounds, platelet receptor antoagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents, vasodilating agents, and agents which interfere with endogenous vasoactive mechanisms;
- anti-oxidants, such as probucol;
- antibiotic agents, such as penicillin, cefoxitin, oxacillin, tobranycin, rapamycin (sirolimus);
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-convertin enzyme (ACE) inhibitors including captropril and enalopril, statins and related compounds; and
- macrolides such as sirolimus or everolimus;

Other therapeutic agents include nitroglycerin, nitrous oxides, nitric oxides, antibiotics, aspirins, digitalis, estrogen, estradiol and glycosides. Exemplary therapeutic agents include anti-proliferative drugs such as steroids, vitamins, and restenosis-inhibiting agents. Exemplary restonosis-inhibiting agents include microtubule stabilizing agents such as Taxol^{®}, paclitaxel (*i.e.*, paclitaxel, paxlitaxel analogs, or paclitaxel derivatives, and mixtures thereof). For example, derivatives suitable for use in the medical devices include 2'-succinyl-taxol, 2'-succinyl-taxol triethanolamine, 2'-glutaryl-taxol, 2'glutaryl-taxol triethanolamine salt, 2'-O-ester with N-(dimethylaminoethyl) glutamine, and 2'-O-ester with N-(dimethylaminoethyl) glutamide hydrochloride salt.

Other exemplary therapeutic agents include tacrolimus; halafuginone; inhibitors of HSP90 heart shock proteins such as geldanamysin; microtubule stabilizing agents such as epothilone D; phosphodiesterase inhibitors such as cliostazole; Barkct inhibitors; phospholamban inhibitors; and Serca 2 gene/proteins. In yet another embodiment, the therapeutic agent is an antibiotic such as erythromycin, amphotericin, rapamycin, adriamycin, etc.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices can be synthesized by methods well known to one skilled in the art. Alternatively, the therapeutic agents can be purchased from chemical and pharmaceutical companies.

### METHODS OF MAKING THE MEDICAL DEVICES

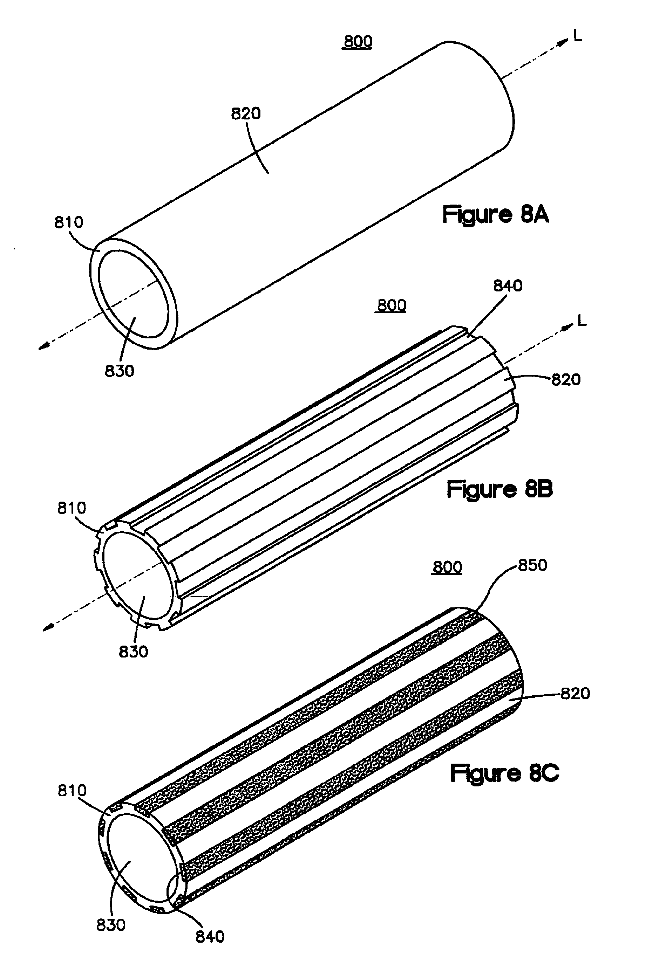

Provided herein are methods of making the medical devices described herein. **Figures 8A-8F** show one embodiment of a method for making an implantable stent. This method comprises providing a tube **800** having a tubular wall **810** and a longitudinal axis **L** as shown in **Figure 8A****.** The tubular wall **810** comprises an abluminal surface **820** and a luminal surface **830.** As shown in **Figure 8B****,** at least one groove **840** is disposed in the abluminal surface **820** of the tubular wall **810.** The grooves **840** do not extend through the tubular wall **810** to the luminal surface **830.** In come embodiments, the method can include the step of forming the groove(s) in the abluminal surface.

The grooves can be formed by various methods, which include without limitation grinding, scoring, or using a laser to remove tube material. Also, the grooves can be formed by any other method known to one skilled in the art, including, but not limited to, sintering, co-deposition, micro-roughing, drilling, chemical etching or a combination thereof. For example, the grooves can be made by a deposition process such as sputtering with adjustments to the deposition condition, by micro-roughening using reactive plasmas, by ion bombardment electrolyte etching, or a combination thereof. Other methods include, but are not limited to, alloy plating, physical vapor deposition, chemical vapor deposition, sintering, or a combination thereof. In addition to material removal techniques, other methods may include incorporating protrusions into a mold used by one skilled in the art for forging the tube or stent. The protrusions create grooves or cavities in the forge tube or stent. Other methods include extruding a tube with grooves already incorporated into the tube.

In the embodiment shown in **Figure 8B****,** the grooves are formed such that they are parallel to the longitudinal axis **L** of the tube **800.** In other embodiments, the grooves can have other configurations such as being circumferential or parallel to the circumference of the tube, *i.e*., perpendicular to the longitudinal axis, being in the shapes of a helix, or in some other desired shape or pattern. The grooves can also be intermittent, *i.e.*, not is a regular pattern or not continuing through the entire tube. The grooves can be of an intermittent longitudinal configuration and/or an intermittent circumferential configuration. The grooves can be intermittent in a configuration such that, upon cutting the stent pattern from the tube, the grooves cross the stent struts. This crossing can be perpendicular to the stent strut.

As shown in **Figure 8C****,** a therapeutic agent **850** is disposed in at least a portion of a groove **840.** In some embodiments, such as the one shown in **Figure 8C****,** the therapeutic agent is disposed in the entire groove. In other embodiments, the therapeutic agent is disposed in less that the entire groove. Such therapeutic agent can include, but are not limited to, any of the therapeutic agents described herein. The therapeutic agent can be disposed in the groove by a micropen process, or a process involving masking and spraying, dipping, roll-cutting, or vapor deposition. The therapeutic agent can also be deposited by a bulk process, such as spray-coating, dip-coating, roll-coating, or vapor deposition, with the agent then removed from the non-grooved surfaces.

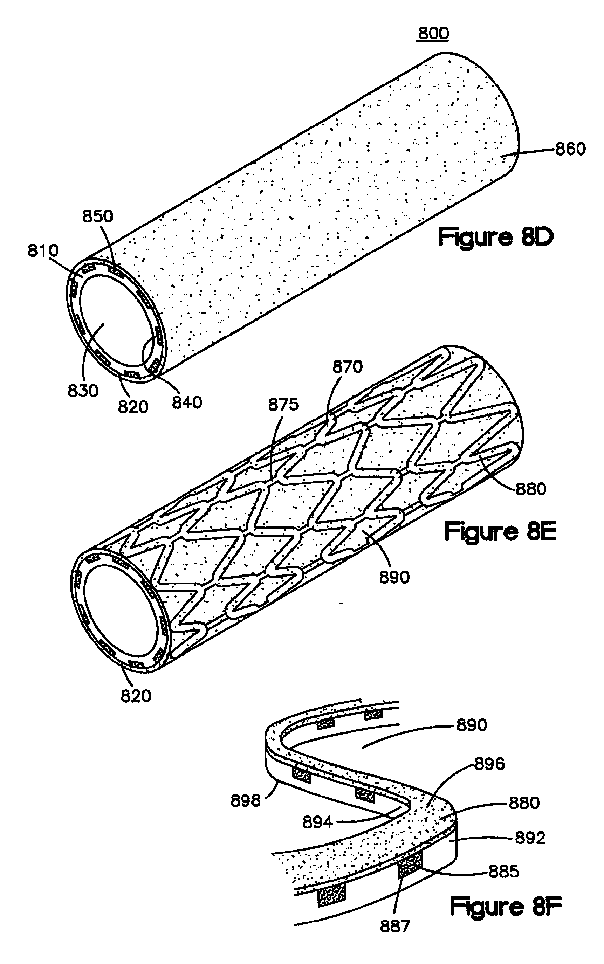

As shown in **Figure 8D****,** a coating **860** is disposed over at least a portion of the abluminal surface **820** of the tubular wall **810** after the therapeutic agent **850** is disposed in the groove **840,** such that at least a portion of the therapeutic agent **850** disposed in the groove **840** is covered by the coating **860.** In some embodiments, all of the therapeutic agent can be covered by the coating while in others less than all the therapeutic agent is covered. In the embodiment shown in **Figure 8D****,** the coating **860** is disposed over the entire abluminal surface **820** of the tubular wall **810.** In other embodiments, the coating is disposed over less than the entire abluminal surface of the tubular wall.

The coating may comprise any of the materials listed herein. In certain embodiments, the coating comprises a material that prevents the release of the therapeutic agent through the coating. In other embodiments, the coating comprises a material having a plurality of pores therein that allows for the release of the therapeutic agent through the coating.

Also, the coating can be disposed on the abluminal surface by methods such as spray-coating, dip-coating, roll coating, or vapor deposition.

As shown in **Figure 8E****,** a stent **870** is formed from the tube **800,** having the coating **860** disposed on the abluminal surface **820.** The stent **870** has a stent sidewall structure **875** comprising a plurality of struts **880** and gaps **890.** As shown in **Figure 8F****,** at least one of the struts **880** comprises an abluminal surface **896,** a luminal surface **898,** a first side surface **892,** a second side surface **894** and a cavity **885.** The cavity **885** comprises a portion of the groove **840** containing the therapeutic agent **850,** and an opening **887** that is in fluid communication with the first side surface **892** and/or second side surface **894.** The opening **887** is at least partially exposed. The stent can be formed by cutting the stent sidewall structure from the tube. In certain embodiments, the cutting can be conducted by using a laser and/or by the use of masking and chemical etching.

Another embodiment of a method for making an implantable stent is similar to the one described above. However, instead of disposing a therapeutic agent in the groove, a filler material is disposed in at least a portion of the groove. After or during the formation of the stent from the tube, at least a portion of the filler material is removed from the cavity of the strut. The filler material can be removed by dissolving the filler material in a solvent. For instance, in one embodiment, the filler material can be dissolved by submerging the stent in a solvent. After at least a port of the filler material is removed, a therapeutic agent is then disposed in the cavity.

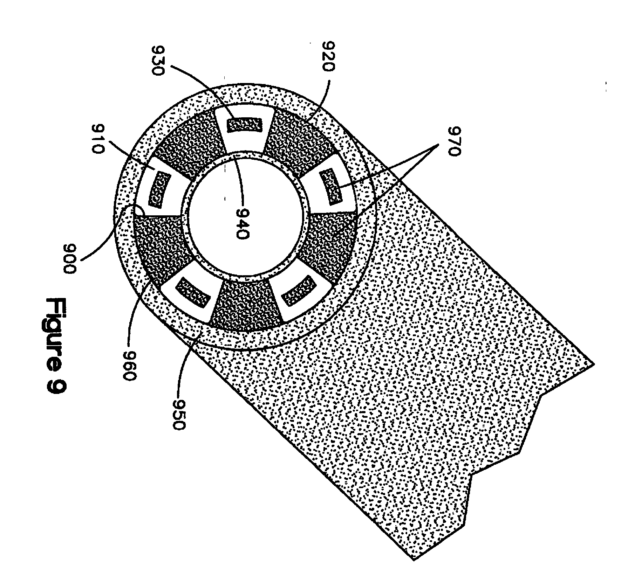

The therapeutic agent is disposed in the cavity in one embodiment by using a mandrel and a tube having an inner wall. In particular, the stent that is formed from the tube, is disposed on an expandable mandrel. The stent disposed on the mandrel is placed in a tube having an inner wall. The mandrel is expanded so that at least a portion of the stent contacts the inner wall of the tube. The stent is then exposed to a therapeutic agent and the therapeutic is allowed to enter the cavity. **Figure 9** shows a cross-sectional view of a stent **900** having struts **910** and gaps **920** disposed between a mandrel **940** and a tube **950** having an inner surface **960.** The struts **910** have cavities **930** therein. In this figure, the stent **900** is exposed to a therapeutic agent **970,** which has entered the cavities **930** and the gaps **920.**

In an alternative embodiment, the stent is disposed on a rigid mandrel. The stent disposed on the mandrel is placed in a tube having an inner wall. Instead of expanding the mandrel, the tube is moved towards or closed around the stent so that at least a portion of the stent contacts the inner wall of the tube. The stent is then exposed to a therapeutic agent and the therapeutic agent is allowed to enter the cavity.

In some instances, the therapeutic agent is contained in a composition that is capable of becoming solid and the stent is exposed to such composition and the composition is allowed to enter the cavity. The composition can be allowed to enter by being pressure forced into the cavity. Also, in some embodiment, the method comprises the step of allowing the composition to solidify or harden.

Furthermore, in some embodiments, the method further comprises removing any excess therapeutic agent from the stent. Such excess therapeutic agent can be removed by laser-cutting, mechanical cutting or water-jet cutting.

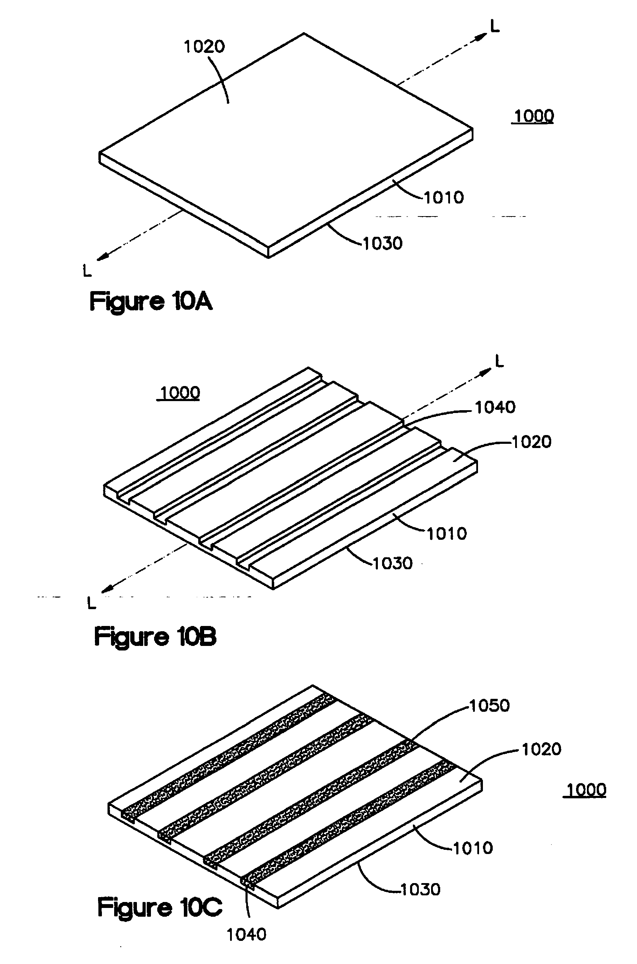

Another embodiment of a method is shown in **Figures 10A-10F****.** This method comprises providing a flat sheet **1000** of a material having a sheet wall of **1010** and a longitudinal axis of **L** as shown in **Figure 10A****.** The sheet wall **1010** comprises a top or first surface **1020** and a bottom or second surface **1030.** As shown in **Figure 10B****,** at least on e groove **1040** is disposed in the first surface **1020** of the tubular wall **1010.** The grooves **1040** do not extend through the sheet wall **1010** to the second surface **1030.** In some embodiments, the method can include the step of forming the groove(s) in the first surface. The grooves can be formed by various methods, including the methods stated herein.

In the embodiment shown in **Figure 10B****,** the grooves **1040** are formed such that they are parallel to the longitudinal axis L of the sheet **1000.** In other embodiments, the grooves can have other configurations such as being perpendicular to the longitudinal axis, forming a checkered pattern on the first surface **1020,** or in some other desired shape, pattern, or configuration such as those stated herein.

As shown in **Figure 10C****,** a therapeutic agent **1050** is disposed in at least a portion of a groove **1040.** In some embodiments, such as the one shown in **Figure 10C**, the therapeutic agent is disposed in the entire groove. In other embodiments, the therapeutic agent is disposed in the less than the entire groove. Such therapeutic agent can include, but are not limited to, any of the therapeutic agents described herein. The therapeutic agent can be disposed in the groove by processes such as those described herein.

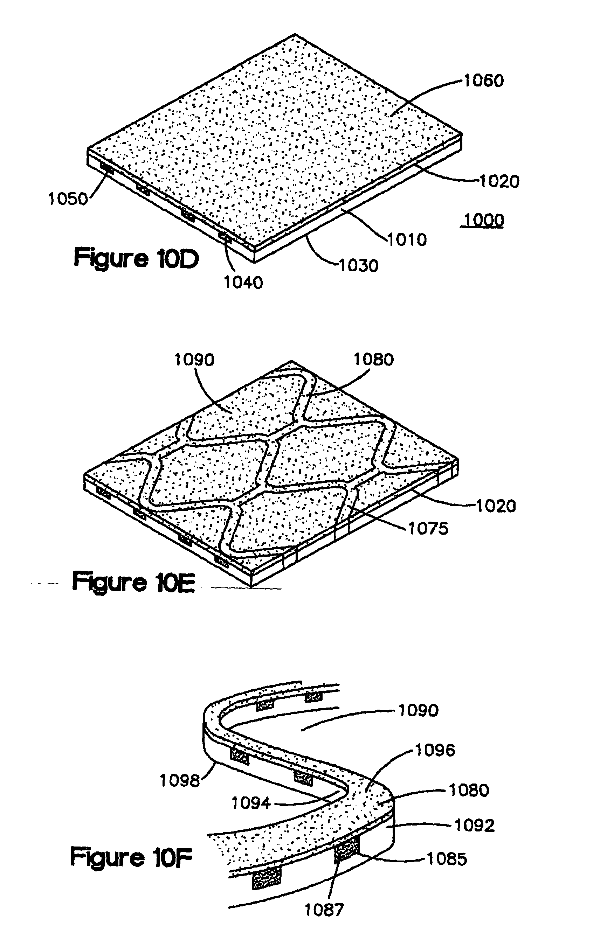

As shown in **Figure 10D****,** a coating **1060** is disposed over at least a portion of the first surface **1020** of the sheet wall **1010** after the therapeutic agent **1050** is disposed in the groove **1040,** such that at least a portion of the therapeutic agent **1050** disposed in the groove **1040** is covered by the coating **1060.** In come embodiments, all of the therapeutic agent can be covered by the coating while in others less than all the therapeutic agent is covered. As shown in **Figure 10D****,** the coating **1060** is disposed over the entire first surface **1020** of the sheet wall **1010.** In other embodiments, the coating is disposed over less than the entire first surface of the sheet wall. The coating may comprise any other material listed herein. Also, the coating can be disposed on the first surface by methods described herein.

As shown in **Figure 10E** a sidewall structure **1075** is formed from the sheet **1000,** having the coating **1060** disposed on the first surface **1020.** After certain portions of the coated sheet are removed, the sidewall structure **1075** comprises a plurality of struts **1080** and gaps **1090** is formed. The sidewall structure can be formed by cutting the sidewall structure from the coated sheet. In certain embodiments, the cutting can be conducted by using a laser and/or by the use of masking and chemical etching.

As shown in **Figure 10F****,** at least one of the struts **1080** of the sidewall structure comprises a top surface **1096,** which can be an abluminal or luminal surface, a bottom surface **1098,** a first side surface **1092,** a second side surface **1094** and a cavity **1085.** The cavity 1085 comprise a portion of the groove **1040** containing the therapeutic agent **1050,** and an opening **1087** that is in fluid communication with the first side surface **1092** and/or second side surface **1094.** The opening **1087** is at least partially exposed. To obtain a tubular shape for a stent, the sheet or sidewall structure can be formed into a tubular shape at any time during the process. In some embodiments, the sidewall structure can be formed into a tubular shape before or after the sidewall structure is formed from the sheet.

Another embodiment of a method for making an implantable stent is similar to the one described above in connection with **Figures 10A-10F****.** However, instead of disposing a therapeutic agent in the groove, a filler material is disposed in at least a portion of the groove. After or during the formation of the sidewall structure from the sheet, at least a portion of the filler material is removed from the cavity of the strut. The filler material can be removed by methods such as those described above in connection with **Figure 9****.** After at least a port of the filler material is removed, a therapeutic agent is then disposed in the cavity.

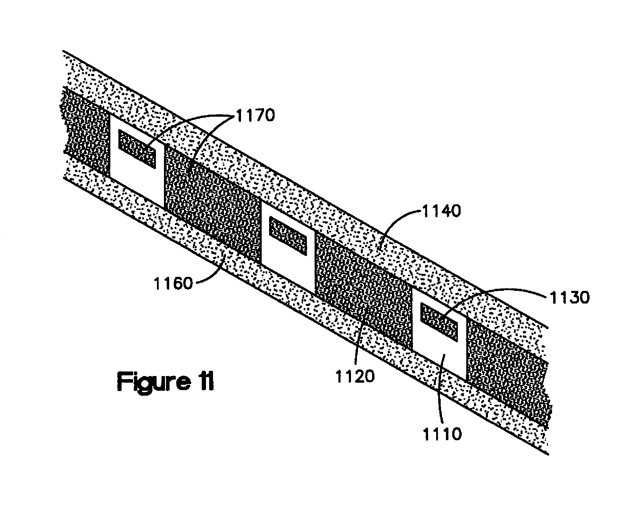

In addition, the therapeutic agent can be disposed in the cavity in one embodiment by using two flat surfaces. In particular, the sidewall structure that is formed from the sheet, is disposed between two flat surfaces. The sidewall structure is then exposed to a therapeutic agent and the therapeutic is allowed to enter the cavity. **Figure 11** shows a cross-sectional view of a sidewall structure having struts **1110** and gaps **1120** disposed between a first plate **1140** and a second plate **1160.** The struts **1110** have cavities **1130** therein. In this figure, the sidewall structure is exposed to a therapeutic agent **1170,** which has entered the cavities **1130** and the gaps **1120.** The first plate **1140** and the second plate **1160** are shown as examples of items that can sandwich the sidewall structure between two surfaces. Various other items, in addition to the plates shown and described herein, will become apparent to those skilled in the art. Also, the sheet or sidewall structure can be formed into a tubular shape at any time during the process.

In another embodiment of a method for making an implantable stent, a therapeutic agent is disposed in the cavity of a strut of a stent after the strut has been formed. The method comprises providing a stent having a stent sidewall structure comprising a plurality of struts and gaps in the sidewall structure. At least one strut comprises an abluminal surface, a luminal surface, a first side surface, and at least one cavity disposed in the strut. The cavity has a first opening that is in fluid communication with the first side surface and second opening that is in fluid communication with the abluminal surface or the luminal surface. A therapeutic agent is disposed in the cavity. Thereafter, a material is applied over the second opening and any other opening so that the cavity is not in fluid communication with the abluminal surface or luminal surface. The first opening which is in fluid communication with the side surface, remains at least partially exposed.

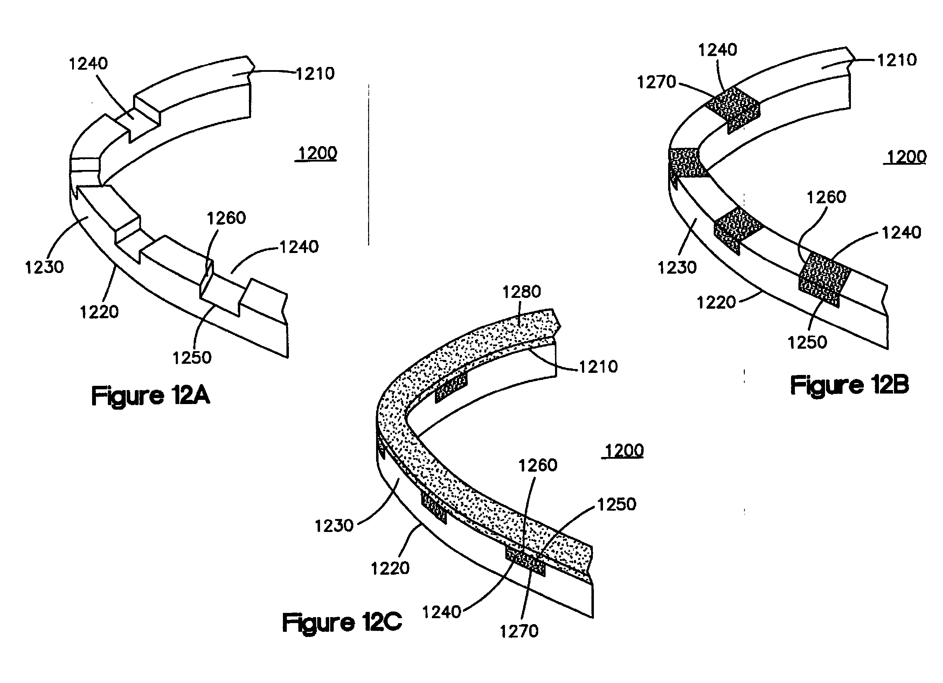

**Figures 12A-12C** show an example of such an embodiment. **Figure 12A** shows a strut **1200** of a stent having an abluminal surface **1210,** luminal surface **1220,** and a first side surface **1230.** The strut comprises at least one cavity **1240** having a first opening **1250** that is in fluid communication with the first side surface **1230** and a second opening **1260** that is in fluid communication with the abluminal surface **1210.** At least a portion of the first opening is in contact with at least a portion of the second opening.

In **Figure 12B****,** a therapeutic agent **1270** is disposed in the cavities **1240.** A material **1280,** which can be the same as or different from the material from which the strut is made, is applied over the second opening **1260** of the cavities **1240,** as shown in **Figure 12C****.** This material **1280** or another material is applied over any other opening of the cavities so that the cavities **1240** are not in fluid communication with the abluminal or luminal surface. The material can comprise those described above in connection with the coating described in **Figure 8D****.** As shown in **Figure 12C****,** the first opening **1250** is at least partially exposed.

In addition, the method can further comprise forming the stent sidewall structure by providing a tube having a tubular wall and a longitudinal axis, in which the tubular wall comprises an abluminal surface and a luminal surface, such as the one shown in **Figure 8A****.** There is at least one groove disposed in the abluminal surface of the tubular wall. The groove does not extend through the tubular wall to the luminal surface. **Figure 8B** shows an example of such a tube having at least one groove. The stent sidewall structure is formed from the tube, such as by cutting the stent sidewall structure from the tube. The struts of the stent sidewall structure will comprise a cavity, which is a portion of the groove.

The method can further comprise forming the stent sidewall structure by providing a flat sheet having a sheet wall and longitudinal axis, in which the sheet wall comprises a first surface and second surface, such as the one shown in **Figure 10A****.** There is at least one groove disposed in the first surface of the sheet wall. The groove does not extend through the sheet wall to the second surface. **Figure 10B** shows an example of such a sheet wall having at least one groove. A sidewall structure is formed from the flat sheet, such as by cutting the sidewall structure from the flat sheet. The struts of the sidewall structure will comprise a cavity, which is a portion of the groove. The flat sheet can be then formed into a tubular shape.

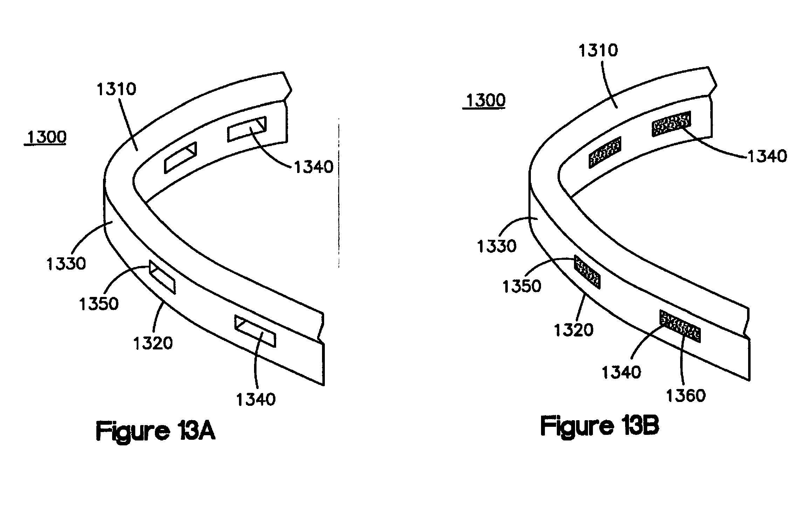

Moreover, another method for making an implantable stent, in which a therapeutic agent is disposed in the cavity of a strut after the strut has been formed, comprises providing a stent having a stent sidewall structure comprising a plurality of struts and gaps in the sidewall structure. **Figure 13A** shows an example of a strut **1300** from such a stent. The strut **1300** comprises an abluminal surface **1310,** a luminal surface **1320,** a first side surface **1330,** and at least one cavity **1340** disposed in the strut **1300.** Each of the cavities **1340** has a first opening **1350** that is in fluid communication with the first side surface **1330** and at least one of the cavities **1340** is not in fluid communication with the abluminal surface **1310** or the luminal surface **1320.** In **Figure 13B****,** a therapeutic agent **1360** is disposed in the cavities **1340;** and the first opening **1350** are allowed to be exposed. In certain embodiments, the formation of the cavities can be conducted by using a laser-cutting or laser-ablation. In certain embodiments, the therapeutic agent is disposed in the cavity by disposing the stent on a mandrel. The stent disposed on the mandrel is placed in a tube having an inner wall. The tube is moved towards or closed around the stent so that at least a portion of the stent contacts the inner wall of the tube. The stent is then exposed to a therapeutic agent and the therapeutic agent is allowed to enter the cavity.

Another embodiment of a method for making an implantable stent comprises disposing a coating on at least one side surface of a stent strut while keeping the abluminal and luminal surfaces of the strut substantially free of the coating composition. This method comprises providing a stent having a stent sidewall structure comprising a plurality of struts and gaps in the sidewall structure, wherein at least one strut comprises an abluminal surface, a luminal surface, and a first side surface. The stent is disposed on a mandrel. A coating composition comprising a therapeutic agent is applied onto a flat surface. The stent disposed on the mandrel is rolled in the coating composition applied to the flat surface to apply the coating composition to the first side surface of the strut.

Steps are taken to ensure that the abluminal surface and the luminal surface are substantially free of the soating composition. In some embodiments, the stent is rolled with sufficient pressure such that the coating composition is applied to the first side surface while the abluminal surface is made substantially free of the coating composition. In other embodiments, the abluminal surface and luminal surface are made substantially free of the coating composition by removing any coating composition disposed on the abluminal surface or luminal surface after the coating composition is applied to the first side surface. The coating composition can be removed by mechanical grinding, laser-ablation, masking and etching, or chemical dissolution of the coating.

The therapeutic agent of the coating composition can be, but are not limited to, those described herein. Also, the coating composition can comprise a polymer, such as but not limited to those described above for forming medical devices. Furthermore, the coating composition can include a solvent for suspending or dissolving the therapeutic agent and/or polymer.

The description contained herein is for purposes of illustration and not for purposes of limitation. The methods and devices described herein can comprise any feature described herein either alone or in combination with any other feature(s) described herein. Changes and modifications may be made to the embodiments of the description. Indeed, various modifications, in addition to those shown and described herein, will become apparent to those skilled in the art from the foregoing description and accompanying drawings using no more than routine experimentation. Such modifications and equivalents are intended to fall within the scope of the appended claims.

## Claims

1. An implantable stent comprising:
a. a stent sidewall structure (100) comprising a plurality of struts (110) and gaps (120) in the sidewall structure (100), wherein at least one strut (110a) comprises an abluminal surface (112), a luminal surface, and a first side surface (114), and wherein the strut (110a) comprises a first material;
b. at least one cavity (130) disposed in the at least one strut (110a), wherein the cavity (130) has a first opening (132) that is in fluid communication with the abluminal surface (112) and a second opening that is in fluid communication with the first side surface (114), wherein the cavity (130) does not extend through the strut (110a) to the luminal surface;
c. a therapeutic agent disposed in the cavity (130); and
d. a coating (140) disposed over at least a portion of the first opening (132) of the cavity (130), wherein the second opening of the cavity (130) is at least partially exposed.

2. The stent of claim 1, wherein at least a portion of the first opening (132) is in contact with at least a portion of the second opening.

3. The stent of claim 1, wherein the strut further comprises a second side surface (116) opposite the first side surface (114) and the cavity has a third opening that is in fluid communication with the second side surface (116); wherein the third opening of the cavity (130) is at least partially exposed.

4. The stent of claim 3, wherein the first or second side surface (114, 116) is a cut-surface.

5. The stent of claim 1, wherein the coating (140) is disposed over the entire first opening (132) of the cavity (130).

6. The stent of claim 1, wherein the coating (140) comprises a material that prevents the release of the therapeutic agent from the cavity (130) through the coating (140).

7. The stent of claim 1, wherein the coating (140) comprises a material having a plurality of pores therein that allows for the release of the therapeutic agent from the cavity (130) through the coating (140).

8. The stent of claim 3, wherein at least a portion of the first opening (132) is in contact with at least a portion of the second opening and at least a portion of the third opening; wherein the therapeutic agent comprises an anti-restenosis agent, and wherein the coating (140) prevents release of the anti-restenosis agent from the cavity (130) through the coating (140).

9. A method for making an implantable stent comprising:
a. providing a tube having a tubular wall having a longitudinal axis, in which the tubular wall comprises an abluminal surface (112) and a luminal surface, and at least one groove disposed in the abluminal surface (112) of the tubular wall, wherein the groove does not extend through the tubular wall to the luminal surface;
b. disposing a filler material in at least a portion of the groove;
c. disposing a coating (110) over at least a portion of the abluminal surface (112) such that at least a portion of the filler material disposed in the groove is covered by the coating (140);
d. forming from the tube, having the coating disposed on the abluminal surface (112), a stent having a stent sidewall structure (100) comprising a plurality of struts (110) and gaps (120), wherein at least one of the struts comprises an abluminal surface (112), a luminal surface, a first side surface (114) and a cavity (130), wherein the cavity (130) comprises a portion of the groove containing the filler material, and an opening that is in fluid communication with the first side surface (114), and wherein the opening is at least partially exposed.
e. removing at least a portion of the filler material from the cavity (130); and
f. disposing a therapeutic agent in the cavity (130).

10. The method of claim 9, wherein the disposing of the therapeutic agent in the cavity comprises:
(1) disposing the stent on an expandable mandrel;
(2) placing the stent disposed on the mandrel in a tube having an inner wall;
(3) expanding the mandrel so that at least a portion of the stent contacts the inner wall of the tube;
(4) exposing the stent to a therapeutic agent; and
(5) allowing the therapeutic agent to enter the cavity (130).

11. The method of claim 10, wherein the therapeutic agent is contained in a composition that is capable of becoming solid and the stent is exposed to the composition and the composition is allowed to enter the cavity (130).

12. The method of claim 10, wherein the composition is allowed to enter the cavity (130) by being pressure forced into the cavity (130).

13. The method of claim 9, wherein the disposing of the therapeutic agent in the cavity (130) comprises:
(1) disposing the stent on an rigid mandrel;
(2) placing the stent disposed on the mandrel in a tube having an inner wall;
(3) closing the tube onto the stent so that at least a portion of the stent contacts the inner wall of the tube;
(4) exposing the stent to a therapeutic agent; and
(5) allowing the therapeutic agent to enter the cavity (130).

## Patentansprüche

1. Implantierbarer Stent umfassend:
a. eine Stent-Seitenwandstruktur (100) umfassend eine Vielzahl von Stützen (110) und Zwischenräumen (120) in der Seitenwandstruktur (100), wobei mindestens eine Stütze (110a) eine abluminale Oberfläche (112), eine luminale Oberfläche und eine erste Seitenfläche (114) umfasst und wobei die Stütze (110a) ein erstes Material umfasst;
b. mindestens einen Hohlraum (130), angeordnet in der mindestens einen Stütze (110a), wobei der Hohlraum (130) eine erste Öffnung (132) aufweist, welche in Fluidkommunikation mit der abluminalen Oberfläche (112) ist, sowie eine zweite Öffnung, welche in Fluidkommunikation mit der ersten Seitenfläche (114) ist, wobei der Hohlraum (130) sich nicht durch die Stütze (110a) hindurch zu der luminalen Oberfläche erstreckt;
c. ein therapeutisches Mittel, angeordnet in dem Hohlraum (130); und
d. eine Beschichtung (140), angeordnet über mindestens einem Teil der ersten Öffnung (132) des Hohlraums (130), wobei die zweite Öffnung des Hohlraums (130) mindestens teilweise frei liegt.

2. Stent gemäß Anspruch 1, wobei mindestens ein Teil der ersten Öffnung (132) in Kontakt mit mindestens einem Teil der zweiten Öffnung ist.

3. Stent gemäß Anspruch 1, wobei die Stütze ferner eine zweite Seitenfläche (116) gegenüber der ersten Seitenfläche (114) umfasst und der Hohlraum eine dritte Öffnung hat, welche in Fluidkommunikation mit der zweiten Seitenfläche (116) ist; wobei die dritte Öffnung des Hohlraums (130) mindestens teilweise frei liegt.

4. Stent gemäß Anspruch 3, wobei die erste oder zweite Seitenfläche (114, 116) eine Schnittfläche ist.

5. Stent gemäß Anspruch 1, wobei die Beschichtung (140) über der gesamten ersten Öffnung (132) des Hohlraums (130) angeordnet ist.

6. Stent gemäß Anspruch 1, wobei die Beschichtung (140) ein Material umfasst, welches die Freisetzung des therapeutischen Mittels aus dem Hohlraum (130) durch die Beschichtung (140) hindurch verhindert.

7. Stent gemäß Anspruch 1, wobei die Beschichtung (140) ein Material mit einer Vielzahl von Poren darin umfasst, welches die Freisetzung des therapeutischen Mittels aus dem Hohlraum (130) durch die Beschichtung (140) hindurch erlaubt.

8. Stent gemäß Anspruch 3, wobei mindestens ein Teil der ersten Öffnung (132) in Kontakt mit mindestens einem Teil der zweiten Öffnung und mindestens einem Teil der dritten Öffnung ist; wobei das therapeutische Mittel ein Mittel gegen Restenose umfasst und wobei die Beschichtung (140) die Freisetzung des Mittels gegen Restenose aus dem Hohlraum (130) durch die Beschichtung (140) hindurch verhindert.

9. Verfahren zum Herstellen eines implantierbaren Stents umfassend:
a. Bereitstellen einer Röhre mit einer röhrenförmigen Wand mit einer Längsachse, bei welcher die röhrenförmige Wand eine abluminale Oberfläche (112) und eine luminale Oberfläche umfasst, sowie mindestens eine in der abluminalen Oberfläche (112) der röhrenförmigen Wand angeordnete Rille, wobei die Rille sich nicht durch die röhrenförmige Wand hindurch zu der luminalen Oberfläche erstreckt;
b. Anordnen eines Füllstoffmaterials in mindestens einem Teil der Rille;
c. Anordnen einer Beschichtung (110) über mindestens einem Teil der abluminalen Oberfläche (112), so dass mindestens ein Teil des in der Rille angeordneten Füllstoffmaterials von der Beschichtung (140) bedeckt ist;
d. Ausbilden aus der Röhre mit der auf der abluminalen Oberfläche (112) angeordneten Beschichtung eines Stents mit einer Seitenwandstruktur (100), die eine Vielzahl von Stützen (110) und Zwischenräumen (120) umfasst, wobei mindestens eine der Stützen eine abluminale Oberfläche (112), eine luminale Oberfläche, eine erste Seitenfläche (114) und einen Hohlraum (130) umfasst, wobei der Hohlraum (130) einen Teil der das Füllstoffmaterial enthaltenden Rille umfasst, sowie eine Öffnung, welche in Fluidkommunikation mit der ersten Oberfläche (114) ist und wobei die Öffnung mindestens teilweise frei liegt.
e. Entfernen von mindestens einem Teil des Füllstoffmaterials aus dem Hohlraum (130); und
f. Anordnen eines therapeutischen Mittels in dem Hohlraum (130).

10. Verfahren gemäß Anspruch 9, wobei das Anordnen des therapeutischen Mittels in dem Hohlraum umfasst:
(1) Anordnen des Stents auf einem ausdehnbaren Dorn;
(2) Verbringen des auf dem Dorn angeordneten Stents in eine Röhre mit einer Innenwand;
(3) Ausdehnen des Dorns, so dass mindestens ein Teil des Stents die Innenwand der Röhre kontaktiert;
(4) Aussetzen des Stents einem therapeutischen Mittel; und
(5) das therapeutische Mittel in den Hohlraum (130) eintreten lassen.

11. Verfahren gemäß Anspruch 10, wobei das therapeutische Mittel in einer Zusammensetzung enthalten ist, welche in der Lage ist, fest zu werden, und der Stent der Zusammensetzung ausgesetzt wird und die Zusammensetzung in den Hohlraum (130) eintreten gelassen wird.

12. Verfahren gemäß Anspruch 9, wobei die Zusammensetzung in den Hohlraum (130) eintreten gelassen wird, indem sie durch Druck in den Hohlraum gezwungen wird.

13. Verfahren gemäß Anspruch 9, wobei das Anordnen des therapeutischen Mittels in dem Hohlraum (130) umfasst:
(1) Anordnen des Stents auf einem steifen Dorn;
(2) Verbringen des auf dem Dorn angeordneten Stents in eine Röhre mit einer Innenwand;
(3) in Anlage bringen der Röhre auf dem Stent, so dass mindestens ein Teil des Stents die Innenwand der Röhre kontaktiert;
(4) Aussetzen des Stents einem therapeutischen Mittel; und
(5) das therapeutische Mittel in den Hohlraum (130) eintreten lassen.

## Revendications

1. Stent implantable comprenant:
a. une structure de paroi latérale du Stent (100) comprenant une pluralité de supports (110) et de trous (120) dans la structure de paroi latérale (100), au moins un support (110a) comprenant une surface abluminale (112), une surface luminale et une première surface latérale (114) et le support (110a) comprenant un premier matériau;
b. au moins une cavité (130), disposée dans l'au moins un support (110a), la cavité (130) ayant une première ouverture (132) qui est en communication fluidique avec la surface abluminale (112), et une deuxième ouverture, qui est en communication fluidique avec la première surface latérale (114), la cavité (130) ne pas s'étendant à travers le support (110a) à la surface luminale;
c. un agent thérapeutique, disposé dans la cavité (130); et
d. un revêtement (140), disposé au-dessus d'au moins une part de la première ouverture (132) de la cavité (130), la deuxième ouverture de la cavité (130) étant au moins partiellement exposée.

2. Stent selon la revendication 1, dans lequel au moins une part de la première ouverture (132) est en contact avec au moins une part de la deuxième ouverture.

3. Stent selon la revendication 1, dans lequel le support comprend en outre une deuxième surface latérale (116) vis-à-vis de la première surface latérale (114) et la cavité a une troisième ouverture, qui est en communication fluidique avec la deuxième surface latérale (116); la troisième ouverture de la cavité (130) étant au moins partiellement exposée.

4. Stent selon la revendication 3, dans lequel la première ou deuxième surface latérale (114, 116) est une surface de cisaillement.

5. Stent selon la revendication 1, dans lequel le revêtement (140) est disposé au-dessus de l'entière première ouverture (132) de la cavité (130).

6. Stent selon la revendication 1, dans lequel le revêtement (140) comprend un matériau qui empêche la libération de l'agent thérapeutique de la cavité (130) à travers le revêtement (140).

7. Stent selon la revendication 1, dans lequel le revêtement (140) comprend un matériau avec une pluralité de pores au-dedans, qui permet la libération de l'agent thérapeutique de la cavité (130) à travers le revêtement (140).

8. Stent selon la revendication 3, dans lequel au moins une part de la première ouverture (132) est en contact avec au moins une part de la deuxième ouverture et avec au moins une part de la troisième ouverture; l'agent thérapeutique comprenant un agent contre la restenose et le revêtement (140) empêchant la libération de l'agent contre la restenose de la cavité (130) à travers le revêtement (140).

9. Procédé pour produire un Stent implantable comprenant:
a. Fournir un tube avec une paroi tubulaire avec un axe longitudinal, dans lequel la paroi tubulaire comprend une surface abluminale (112) et une surface luminale, et au moins une rainure disposée dans la surface abluminale (112) de la paroi tubulaire, la rainure ne pas s'étendant à la surface luminale à travers la paroi tubulaire;
b. disposer une matière de remplissage dans au moins une part de la rainure;
c. disposer un revêtement (110) au-dessus d'au moins une part de la surface abluminale (112), de sorte que au moins une part de la matière de remplissage disposée dans la rainure est recouverte par le revêtement (140);
d. former à partir du tube, avec revêtement disposé sur la surface abluminale (112), un Stent avec une structure de paroi latérale du Stent (100), qui comporte une pluralité de supports (110) et de trous (120), dans lequel au moins un des supports comprend une surface abluminale (112), une surface luminale, une première surface latérale (114) et une cavité (130), la cavité (130) comprenant une part de la rainure qui contient la matière de remplissage et une ouverture qui est en communication fluidique avec la première surface (114) et dans lequel l'ouverture est au moins partiellement exposée.
e. éliminer au moins une part de la matière de remplissage de la cavité (130); et
f. mettre en place un agent thérapeutique dans la cavité (130).

10. Procédé selon la revendication 9, dans lequel la mise en place de l'agent thérapeutique dans la cavité comprend:
(1) disposer le Stent sur un mandrin dilatable;
(2) mettre le Stent disposé sur le mandrin dans un tube avec une paroi intérieure;
(3) dilater le mandrin, de sorte que au moins une part du Stent contacte la paroi intérieure du tube;
(4) exposer le Stent à un agent thérapeutique; et
(5) laisser l'agent thérapeutique entrer dans la cavité (130).

11. Procédé selon la revendication 10, dans lequel l'agent thérapeutique est contenu dans une composition qui est capable à devenir solide, et le Stent est exposé à la composition et la composition est permise à entrer dans la cavité (130).

12. Procédé selon la revendication 9, dans lequel la composition est permise à entrer dans la cavité (130) en étant forcée au-dedans de la cavité par pression.

13. Procédé selon la revendication 9, dans lequel la mise en place de l'agent thérapeutique dans la cavité comprend:
(1) disposer le Stent sur un mandrin rigide;
(2) mettre le Stent disposé sur le mandrin dans un tube avec une paroi intérieure;
(3) fermer le tube sur le Stent, de sorte que au moins une part du Stent contacte la paroi intérieure du tube;
(4) exposer le Stent à un agent thérapeutique; et
(5) laisser l'agent thérapeutique entrer dans la cavité (130).
